# EUROPEAN PATENT APPLICATION

(11) **EP 4 548 982 A1**
(43) Date of publication of application: **07.05.2025**
(21) Application number: 22969468.2
(22) Date of filing: 26.12.2022
(51) Int. Cl.: A63B 24/00, A63B 71/06

(54) **MOTION DATA PROCESSING METHOD AND SYSTEM**

(71) Applicant: Shenzhen Shokz Co., Ltd., Shenzhen, Guangdong 518108 (CN)
(72) Inventor: ZHOU, Xin, Shenzhen, Guangdong 518108 (CN)
(74) Representative: Wang, Bo
(86) International application number: PCT/CN2022/141898
(87) International publication number: WO 2024/138311

(57) **Abstract**

One or more embodiments of the present disclosure relates to a method and system for processing motion data. The method includes obtaining motion data of a user at different stride frequencies, the motion data at least including electromyographic (EMG) signals; determining a physiological state of the user based on the motion data; and determining a recommended stride frequency for the user based on the physiological state.

## Description

### TECHNICAL FIELD

The present disclosure relates to the technical field of data collection and processing, and in particular, to a method and system for processing motion data.

### BACKGROUND

Scientific running becomes increasingly important as people pay more attention to their health. A stride frequency and a stride length of running are used to measure whether the people are running scientifically. Generally, the stride length is closely related to the height, leg length, joint flexibility, core strength, etc. of a person. In contrast, the stride frequency is easier to change through training, thereby improving the exercise effect of running. A current method for determining the stride frequency is to use a standard stride frequency of running athletes (e.g., 180 steps per minute) as a recommended running stride frequency for the general population, whereas the stride frequency of the running athletes does not apply to every general population, considering differences in fitness between the general population and the athletes, as well as differences in physical fitness between the general populations.

Therefore, it is particularly important to determine an appropriate stride frequency for users to help them perform running motions more safely and scientifically.

### SUMMARY

Embodiments of the present disclosure provide a method for processing motion data, including: obtaining motion data of a user at different stride frequencies, the motion data at least including electromyographic (EMG) signals; determining a physiological state of the user based on the motion data; and determining a recommended stride frequency for the user based on the physiological state.

In some embodiments, the obtaining the motion data of a user at different stride frequencies includes: determining a target speed interval; and obtaining the motion data at different stride frequencies within the target speed interval based on the target speed interval

In some embodiments, the physiological state includes a muscle efficiency, a muscle tension, and a muscle fatigue, and the determining a recommended stride frequency for the user based on the physiological state includes: determining a correspondence between physiological states and stride frequencies based on at least one of the muscle efficiency, the muscle tension, and the muscle fatigue; and determining the recommended stride frequency for the user based on the correspondence.

In some embodiments, the determining a physiological state of the user based on the motion data includes: determining at least one of the muscle efficiency, the muscle tension, and the muscle fatigue of the user based on the EMG signals.

In some embodiments, the motion data includes electrocardio (ECG) signals and/or posture signals, and the determining a recommended stride frequency for the user based on the physiological state includes: determining a correspondence between physiological states and stride frequencies based on the ECG signals and/or the posture signals; and determining the recommended stride frequency for the user based on the correspondence.

In some embodiments, the determining the physiological state of the user based on the motion data includes: determining the physiological state of the user based on the ECG signals and/or the posture signals, the physiological state including an injury risk for the motion of the user.

In some embodiments, the obtaining motion data of a user at different stride frequencies includes: obtaining a real-time stride frequency and real-time motion data of the user; dividing the real-time stride frequency into a plurality of stride frequency intervals; and determining, based on the plurality of stride frequency intervals, the motion data within each different stride frequency interval from the real-time motion data.

In some embodiments, the real-time stride frequency is obtained by an inertial sensor, or the real-time stride frequency is obtained from the EMG signals.

In some embodiments, the method further includes: feeding back the recommended stride frequency to the user.

In some embodiments, the feeding back the recommended stride frequency to the user includes: obtaining a real-time stride frequency of the user; determining a stride frequency adjustment trend for the user based on a frequency difference between the real-time stride frequency and the recommended stride frequency; and guiding the user based on the stride frequency adjustment trend until a stride frequency of the user is the same as the recommended stride frequency.

In some embodiments, the feeding back the recommended stride frequency to the user includes: feeding back a beat that is the same as the recommended stride frequency to the user based on a preset feedback manner.

In some embodiments, the preset feedback manner includes at least one of a voice feedback, a vibration feedback, a light feedback, and a display feedback.

In some embodiments, the feeding back the recommended stride frequency to the user includes: stimulating a body portion of the user based on a biofeedback manner to feed back the recommended stride frequency to the user. The stimulation has a stimulation frequency same as the recommended stride frequency.

In some embodiments, the obtaining motion data of a user at different stride frequencies includes: obtaining a plurality of sets of motion data of the user at a plurality of preset stride frequencies, each of the plurality of sets of motion data corresponding to one of the plurality of preset stride frequencies.

In some embodiments, the obtaining a plurality of sets of motion data of the user at a plurality of preset stride frequencies includes: obtaining physiological information of the user, the physiological information including body information and motion information; and determining the plurality of sets of motion data of the user at the plurality of preset stride frequencies based on the physiological information.

Embodiments of the present disclosure also provide a system for processing motion data including: an obtaining module configured to obtain motion data of a user at different stride frequencies, the motion data at least including electromyographic (EMG) signals; a processing module configured to determine a physiological state of the user based on the motion data; and a recommendation module configured to determine a recommended stride frequency for the user based on the physiological state.

In some embodiments, the system further includes a feedback module configured to feed back the recommended stride frequency to the user.

Embodiments of the present disclosure also provide a wearing body provided with at least one sensor, the at least one sensor being configured to obtain motion data of a user; and a processor configured to perform the method for processing motion data in any embodiment of the present disclosure.

In some embodiments, the wearing body is further provided with electrodes contacting a skin of the user, and the electrodes are configured to provide stimulation to a body portion of the user.

### BRIEF DESCRIPTION OF THE DRAWINGS

The present disclosure is further illustrated by way of exemplary embodiments, which is described in detail by means of the accompanying drawings. These embodiments are not limiting, and in these embodiments, the same numbering denotes the same structure, wherein:
FIG. 1 is a schematic diagram illustrating an exemplary system for processing motion data according to some embodiments of the present disclosure;
FIG. 2 is a block diagram illustrating an exemplary device for processing motion data according to some embodiments of the present disclosure;
FIG. 3 is a flowchart illustrating an exemplary process for processing motion data according to some embodiments of the present disclosure;
FIG. 4 is a flowchart illustrating an exemplary process for obtaining motion data according to some embodiments of the present disclosure;
FIG. 5 is a flowchart illustrating an exemplary process for determining a recommended stride frequency according to some embodiments of the present disclosure; and
FIG. 6 is a flowchart illustrating an exemplary process for feeding back a recommended stride frequency according to some embodiments of the present disclosure.

### DETAILED DESCRIPTION

To more clearly illustrate the technical solutions of the embodiments of the present disclosure, the following description of the accompanying drawings required to be used in the description of the embodiments are briefly described. Obviously, the accompanying drawings in the following description are only some examples or embodiments of the present disclosure, and it is possible for those skilled in the art to apply the present disclosure to other similar scenarios in accordance with these drawings without creative labor. Unless obviously obtained from the context or the context illustrates otherwise, the same numeral in the drawings refers to the same structure or operation.

It should be understood that the terms "system," "device," "unit," and/or "module" are used herein as a way to distinguish between different components, elements, portions, sections, or assemblies at different levels. However, the words may be replaced by other expressions if other words accomplish the same purpose.

As shown in the present disclosure and in the claims, unless the context clearly suggests an exception, the words "one," "a," "an," and/or "the" do not refer specifically to the singular, but may also include the plural. Generally, the terms "including" and "comprising" suggest only the inclusion of clearly identified operations and elements. In general, the terms "including" and "comprising" only suggest the inclusion of explicitly identified operations and elements that do not constitute an exclusive list, and the method or device may also include other operations or elements.

Flowcharts are used in the present disclosure to illustrate operations performed by a system in accordance with embodiments of the present disclosure. It should be appreciated that the preceding or following operations are not necessarily performed in an exact sequence. Instead, operations are processed in reverse order or simultaneously. Also, it is possible to add other operations to these processes or remove a step or steps from them.

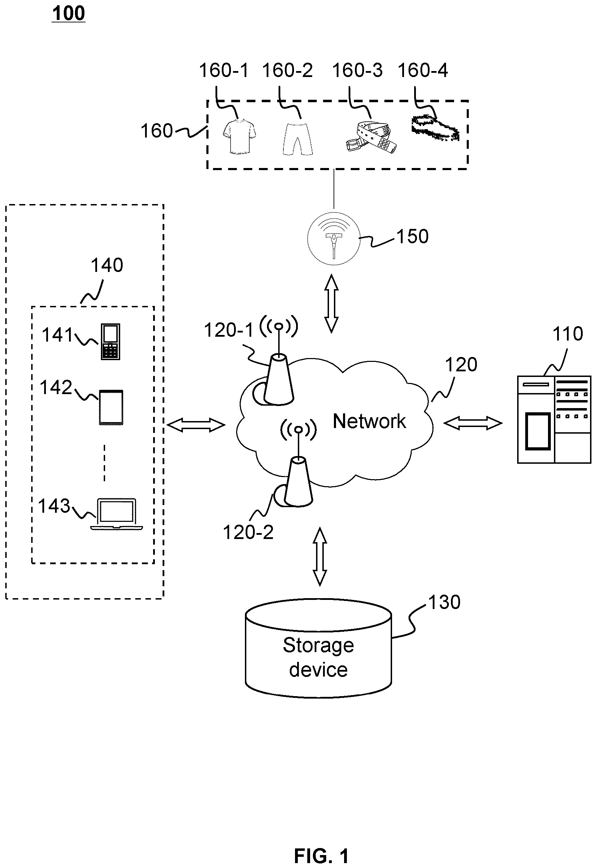

FIG. 1 is a schematic diagram illustrating an exemplary system for processing motion data according to some embodiments of the present disclosure. As shown in FIG. 1, a system for processing motion data 100 includes a processing device 110, a network 120, a storage device 130, a terminal device 140, and a data collection device 150. Components in the system for processing motion data 100 are connected in various manners. For example, the data collection device 150 is connected to the storage device 130 and/or the processing device 110 via the network 120, or directly connected to the storage device 130 and/or the processing device 110. As another example, the storage device 130 is directly connected to the processing device 110 or connected via the network 120. As another example, the terminal device 140 is connected to the storage device 130 and/or the processing device 110 via the network 120 or is directly connected to the storage device 130 and/or the processing device 110. In some embodiments, the system for processing motion data 100 also includes a wearable device 160, with the data collection device 150 disposed on the wearable device 160 (e.g., a top 160-1, pants 160-2, a belt 160-3). The wearable device 160 is connected to various components of the system for processing motion data 100 via the data collection device 150.

In some embodiments, the system for processing motion data 100 determines a recommended stride frequency for a user while running by implementing the methods and/or processes disclosed in the present disclosure. For example, when the user wears the wearable device 160 and runs, the data collection device 150 fits on a human skin to collect motion data of the user while running. The processing device 110 directly or indirectly obtains the motion data (e.g., electromyographic (EMG) signals, posture signals, electrocardio (ECG) signals, etc.) from the data collection device 150 and determines a physiological state of the user based on the motion data, thereby determining the recommended stride frequency for the user when running.

The processing device 110 processes data and/or information obtained from the data collection device 150, the storage device 130, the terminal device 140, and/or other components of the system for processing motion data 100. In some embodiments, the processing device 110 obtains the motion data of the user from any one or more of the data collection device 150, the storage device 130, or the terminal device 140, and determines the recommended stride frequency for the user by processing the motion data. In some embodiments, the processing device 110 obtains pre-stored computer instructions from the storage device 130 and performs the computer instructions to implement a method for processing the motion data described herein.

In some embodiments, the processing device 110 is a single server or a server group. The server group is centralized or distributed. In some embodiments, the processing device 110 is local or remote. For example, the processing device 110 accesses the information and/or data from the data collection device 150, the storage device 130, and/or the terminal device 140 via the network 120. As another example, the processing device 110 is directly connected to the data collection device 150, the storage device 130, and/or the terminal device 140 to access the information and/or data. In some embodiments, the processing device 110 is implemented on a cloud platform. For example, the cloud platform includes a private cloud, a public cloud, a hybrid cloud, a community cloud, a distributed cloud, an internal cloud, a multi-layer cloud, etc., or any combination thereof.

The network 120 connects various components of the system for processing motion data 100 and/or connects the system for processing motion data 100 to an external resource portion. The network 120 enables communications between components of the system for processing motion data 100, and between other portions apart from the system for processing motion data 100, to facilitate an exchange of the data and/or information. For example, the processing device 110 obtains the motion data from the data collection device 150 and/or the storage device 130 via the network 120. As another example, the processing device 110 controls the terminal device 140 to feed back the recommended stride frequency via the network 120 to the user. Exemplary feedback manners include at least one of a voice feedback, a vibration feedback, a light feedback, a display feedback, etc.

In some embodiments, the network 120 is any form of wired or wireless network, or any combination thereof. Merely by way of example, the network 120 includes a cable network, a wired network, a fiber optic network, a telecommunication network, an intranet, the Internet, a local area network (LAN), a wide area network (WAN), a wireless local area network (WLAN), a metropolitan area network (MAN), a public switched telephone network (PSTN), a Bluetooth network, a ZigBee network, a near-field communication (NFC) network, etc., or any combination thereof. In some embodiments, the network 120 includes one or more network access points. For example, the network 120 includes wired or wireless network access points, such as base stations and/or inter-network switching points 120-1, 120-2, ..., through which one or more components of the system for processing motion data 100 are connected to the network 120 to exchange the data and/or information. For example, data such as the motion data, the physiological states of a user, etc. is communicated via the network 120.

The storage device 130 stores data, instructions, and/or any other information. In some embodiments, the storage device 130 stores the data obtained from the data collection device 150, the processing device 110, and/or the terminal device 140. For example, the storage device 130 stores the motion data collected by the data collection device 150. In some embodiments, the storage device 130 stores the data and/or instructions used by the processing device 110 to accomplish the exemplary methods described in the present disclosure. In some embodiments, the storage device 130 includes a mass memory, a removable memory, a volatile read-write memory, a read-only memory (ROM), etc., or any combination thereof. Exemplary mass storages includes disks, optical disks, solid state disks, etc. In some embodiments, the storage device 130 is implemented on the cloud platform. Merely by way of example, the cloud platform includes the private cloud, the public cloud, the hybrid cloud, the community cloud, the distributed cloud, an internal cloud, a multi-layer cloud, etc., or any combination thereof.

In some embodiments, the storage device 130 is connected to the network 120 to communicate with at least one other component (e.g., the data collection device 150, the processing device 110, the terminal device 140) of the system for processing motion data 100. At least one component of the system for processing motion data 100 accesses the data, the instructions, or other information stored in the storage device 130 via the network 120. In some embodiments, the storage device 130 is directly connected or in communication with one or more components (e.g., the data collection device 150, the terminal device 140) of the system for processing motion data 100. In some embodiments, the storage device 130 is a portion of the data collection device 150 and/or the processing device 110.

The terminal device 140 receives, sends, and/or displays data. The data received by the terminal device 140 includes data collected by the data collection device 150, data stored by the storage device 130, the recommended stride frequency generated by the processing device 110, etc. For example, the data received and/or displayed by the terminal device 140 includes the motion data collected by the data collection device 150, physiological state data of the user obtained by processing the motion data by the processing device 110, the recommended stride frequency determined by the processing device 110 based on the physiological state, etc. The data sent by the terminal device 140 includes user input data and instructions, etc. For example, the terminal device 140 sends the user input operation instructions to the data collection device 150 via the network 120 to control the data collection device 150 to perform corresponding data collection (e.g., collecting EMG signals, ECG signals, posture signals, etc.).

In some embodiments, the terminal device 140 includes a mobile device 141, a tablet computer 142, a laptop computer 143, etc., or any combination thereof. In some embodiments, the mobile device 141 includes a mobile phone, a smart mobile device, a virtual reality (VR) device, an augmented reality (AR) device, etc., or any combination thereof. In some embodiments, the smart mobile device includes a smart phone, a personal digital assistant (PDA), a gaming device, a navigation device, a point of sales (POS) device, etc., or any combination thereof. In some embodiments, the VR device and/or the AR device includes a VR headset, VR glasses, a VR eyepatch, an AR headset, AR glasses, an AR eyepatch, etc., or any combination thereof. In some embodiments, the processing device 110 is a portion of the terminal device 140.

It is noted that the system for processing motion data 100 may not include the terminal device 140, and functions or portions of the functions of the terminal device 140 are performed by the wearable device 160. For example, the wearable device 160 is used to control the data collection device 150 to perform the corresponding data collection. As another example, data (e.g., the recommended stride frequency) displayed to the user via the terminal device 140 is displayed by the wearable device 160 (e.g., the wearable device 160 feeds back the recommended stride frequency in the form of electrical stimulation to the user).

The data collection device 150 is a device that performs motion data collection on the user. The motion data refers to signals generated by the user while running. Exemplary motion data includes one or more of the EMG signals, the posture signals, the ECG signals, respiratory signals, sweat signals, mechanical signals, etc. In some embodiments, the data collection device 150 includes an EMG data collection device, a posture data collection device, and a mechanical data collection device. In some embodiments, the EMG data collection device includes one or more electrodes. For example, the EMG data collection device includes a plurality of electrodes disposed at different positions of the wearable device 160 for fitting to different portions of the user (e.g., a chest, a back, elbows, legs, abdomen, wrists, etc.) to collect the EMG signals from the different portions of the user. In some embodiments, the posture data collection device includes a velocity sensor, an inertial sensor (e.g., an acceleration sensor, an angular velocity sensor (e.g., a gyroscope), etc.), an optical sensor (e.g., an optical distance sensor, a video/image grabber), an acoustic distance sensor, a tension sensor, etc., or any combination thereof. In some embodiments, the mechanical data collection device includes a pressure sensor. For example, the pressure sensor is provided at different portions of the user to collect pressure signals at different portions. In some embodiments, the data collection device 150 also includes an ECG data collection device, a respiratory data collection device, a sweat data collection device, etc. For example, the ECG data collection device includes a plurality of electrodes, and the plurality of electrodes are used to fit different portions of the user (e.g., to the abdomen via the belt device 160-3) to collect the ECG signals from the user. As another example, the respiratory data collection device includes a respiratory frequency sensor, a flow rate sensor, etc. for detecting signals such as a respiratory frequency, a gas flow rate, etc., respectively, of the user during exercise. As a further example, the sweat data collection device includes a plurality of electrodes that are in contact with a user's skin for detecting a sweat flow of the user and analyzing the composition of the sweat, etc. In some embodiments, the data collection device 150 has an independent power supply and sends the collected data to other components (e.g., the processing device 110, the storage device 130, the terminal device 140) via wired or wireless (e.g., Bluetooth, WiFi, etc.) manners.

The wearable device 160 refers to a wearable garment or device. In some embodiments, the wearable device 160 includes, but is not limited to, a top device 160-1, a pants device 160-2, a belt device 160-3, a shoe device 160-4, etc. In some embodiments, one or more data collection devices 150 are disposed in the wearable device 160 to collect the motion data from different portions of the body. When the user wears the wearable device 160 and exercises, the one or more data collection devices 150 are in contact with body portions of the user to collect the motion data. For example, the one or more data collection devices 150 (e.g., the electrodes) on the wearable device 160 are in contact with a plurality of portions of the human body, e.g., calves, thighs, hips, the waist, the chest, shoulders, etc., to obtain the EMG signals from the portions of the body. As another example, the one or more data collection devices 150 (e.g., the inertial sensor) on the wearable device 160 are in contact with a plurality of limbs (e.g., upper arms, forearms, thighs, calves, etc.) or joint portions (e.g., knee joints, ankles, elbow joints, etc.) of the human body to obtain the posture signals from the corresponding portions. In some embodiments, devices such as the top device 160-1, the pants device 160-2, the belt device 160-3, and the shoe device 160-4 serve as a wearing body of the wearable device 160, and the data collection device 150 is disposed on the wearing body.

It is noted that the wearing body of the wearable device 160 is not limited to the top device 160-1, the pants device 160-2, the belt device 160-3, and the shoe device 160-4 shown in FIG. 1, but also includes other devices, such as a wristband device, a glove device, etc., which are not specifically limited herein. It is appreciated that the user who wears the wearable device 160 and runs may be a runner, a tester of a sports device, etc. The count of the users can be one or more.

The description of the system for processing motion data 100 is intended to be illustrative and is not to limit the scope of the present disclosure. Numerous substitutions, modifications, and variations are apparent to those skilled in the art. It is appreciated that for those skilled in the art, when knowing a principle of the system, without departing from the principle, any combination of modules is made, or subsystems are formed to connect with other modules. The features, structures, methods, and other characteristics of the exemplary embodiments described in the present disclosure are combined in various ways to obtain additional and/or alternative exemplary embodiments. These changes are within the scope of protection of the present disclosure.

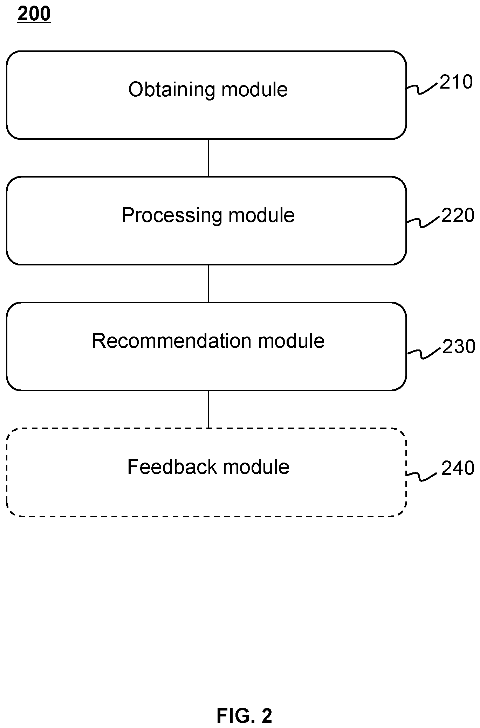

FIG. 2 is a block diagram illustrating an exemplary device for processing motion data according to some embodiments of the present disclosure. In some embodiments, a device for processing motion data 200 shown in FIG. 2 is applied to the system for processing motion data 100 shown in FIG. 1, e.g., the device for processing motion data 200 is configured to the processing device 130 and/or the terminal device 140 in a form of software and/or hardware and applied in determining a recommended stride frequency for a user based on motion data of the user. In some embodiments, the device for processing motion data 200 includes an obtaining module 210, a processing module 220, a recommendation module 230, and a feedback module 240.

The obtaining module 210 may be configured to obtain the motion data of the user at different stride frequencies. For example, the obtaining module 210 obtains the motion data from any one or more of the data collection device 150, the storage device 130, or the terminal device 140. In some embodiments, the motion data includes EMG signals, posture signals, ECG signals, respiratory signals, sweat signals, mechanical signals, etc.

The processing module 220 may be configured to determine a physiological state of the user based on the motion data. In some embodiments, the physiological state includes a muscle efficiency, a muscle tension, a muscle fatigue, an injury risk for the motion, etc. In some embodiments, the processing module 220 determines at least one of the muscle efficiency, the muscle tension, and the muscle fatigue of the user based on the EMG signals, thereby determining the physiological state of the user. In some embodiments, the processing module 220 determines the injury risk of the motion for the user based on the ECG signal and/or the posture signal, thereby determining the physiological state of the user.

The recommendation module 230 may be configured to determine a recommended stride frequency for the user based on the physiological state. In some embodiments, the recommendation module 230 is configured to determine the recommended stride frequency for the user based on a correspondence between physiological states and stride frequencies. For example, the recommendation module 230 determines the recommended stride frequency based on a correspondence between the muscle efficiency, the muscle tension, and the muscle fatigue, and the stride frequencies. As another example, the recommendation module 230 determines the recommended stride frequency based on the correspondence between the injury risk for the motion and the stride frequencies.

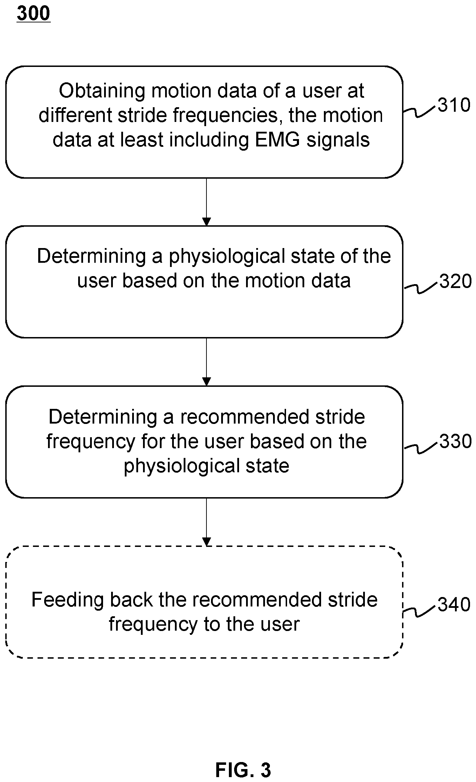

The feedback module 240 may be configured to feed back the recommended stride frequency to the user. In some embodiments, the feedback module 240 feeds back a beat that is the same as the recommended stride frequency to the user based on a preset feedback manner. (e.g., a voice feedback, a vibration feedback, etc.). In some embodiments, the feedback module 240 guides the user to adjust the stride frequency based on a real-time stride frequency of the user until the stride frequency of the user is the same as the recommended stride frequency. In some embodiments, a feedback manner of the feedback module 240 is a real-time feedback. The user views or receives feedback results in real time via a terminal device (e.g., a cell phone, a watch). In some embodiments, the feedback manner of the feedback module 240 is non-real-time feedback. The user views statistics of a motion situation generated after the motion via the terminal device (e.g., the cell phone, the watch, a computer). In some embodiments, the feedback module 240 is configured to provide feedback to the user on the recommended stride frequency, and the feedback module 240 may also be omitted, with components of other modules (e.g., the obtaining module 210) performing a feedback function. More details regarding the various modules of the device for processing motion data 200 may be found in FIG. 3 - FIG. 6 of the present disclosure and the related descriptions.

It should be noted that the above descriptions of the device for processing motion data 200 and the modules are provided only for descriptive convenience, and do not limit the present disclosure to the scope of the cited embodiments. It is to be understood that, for those skilled in the art, with an understanding of the principle of the device and/or the modules, it may be possible to arbitrarily combine the individual modules, or form sub-modules to connect to the other modules, without departing from the principle. For example, the feedback module 240 is omitted or substituted with other devices or modules, and such variations are within the scope of protection of the present disclosure.

FIG. 3 is a flowchart illustrating an exemplary process for processing motion data according to some embodiments of the present disclosure. In some embodiments, a process 300 is executed according to a processing logic that includes a hardware (e.g., a circuitry, a specialized logic, a programmable logic, a microcode, etc.), software (running on a processing device to execute instructions simulated by the hardware), etc., or any combination thereof. In some embodiments, one or more operations in the process 300 for processing motion data illustrated in FIG. 3 are implemented by the processing device 110 and/or the terminal device 140 illustrated in FIG. 1. For example, the process 300 is stored in a form of an instruction in the storage device 130 and is invoked and/or executed by the processing device 110 and/or the terminal device 140. As shown in FIG. 3, the process 300 includes the following operations.

In 310, motion data of a user at different stride frequencies is obtained, the motion data at least including EMG signals.

In some embodiments, operation 310 is performed by the obtaining module 210. In some embodiments, the motion data is data generated by the user during running. The motion data is used to characterize a state (e.g., a physiological state) of the user during running. In some embodiments, the motion data includes the EMG signals. The EMG signals are bioelectrical signals generated by muscles during a motion process. The EMG signals are used to determine the physiological state of the user during running (e.g., a muscle efficiency, a muscle tension). In some embodiments, when the user wears a wearable device, the EMG signals are obtained by a data collection device (e.g., one or more electrodes) fitted to the user. For example, the one or more electrodes are fitted to the muscles in different portions of the user (e.g., thighs, calves, waist, buttocks) to collect the EMG signals from the corresponding muscle portions.

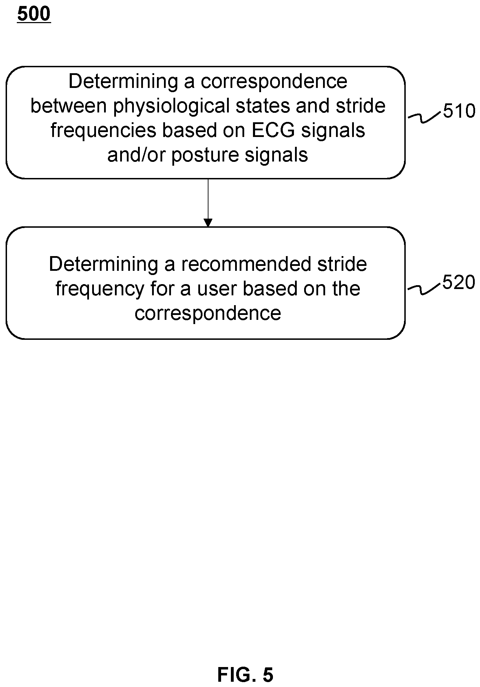

In some embodiments, the motion data also includes posture signals and the ECG signals. The posture signals include information such as angles, velocities, and accelerations of various joints, or Euler angles, angular velocities, and angular accelerations of various body portions. In some embodiments, the posture signals are used to characterize the physiological state of a current motion (e.g., injury risk for the motion) of the user. In some embodiments, the posture signals are obtained by the data collection device (e.g., a posture data collection device). Exemplary posture data collection devices include a velocity sensor, an inertial sensor (e.g., an acceleration sensor, an angular velocity sensor (e.g., a gyroscope), etc.), an optical sensor (e.g., an optical distance sensor, a video/image grabber), an acoustic distance sensor, a tension sensor, etc., or any combination thereof. The ECG signals refer to signals used to indicate a heart activity of the user. In some embodiments, the ECG signals are collected by an ECG data collection device. For example, the ECG data collection device includes a plurality of electrodes. The plurality of electrodes are used to fit different portions of the user, for example, an abdomen, for obtaining the ECG signals of the user. More descriptions regarding the posture signals and the ECG signals may be found in FIG.5 of the present disclosure and the related descriptions.

In other embodiments, the motion data also includes respiratory signals, mechanical signals, sweat signals, etc., or any combination thereof. The respiratory signals refer to signals used to indicate the respiratory of the user. In some embodiments, the respiratory signals are collected by a respiratory data collection device. For example, the respiratory data collection device includes a respiratory frequency sensor, a flow rate sensor, etc. for detecting data such as a respiratory frequency, a gas flow rate, etc., respectively, of the user during motion. The sweat signals refer to signals used to indicate a sweating of the user. In some embodiments, the sweat signals are collected by a sweat data collection device. For example, the sweat data collection device includes a plurality of electrodes contacting the skin of the user for detecting a flow of sweat or analyzing a composition of the sweat of the user, etc. The mechanical signals refer to corresponding forces at the joints of the user, and the mechanical signals are used to characterize the injury risk for the motion of the user (e.g., a pressure at ankles, a pressure at knees, etc.). In some embodiments, the mechanical signals are obtained by a mechanical sensor. For example, the mechanical sensor includes a pressure sensor, and pressure signals from different portions of the user are obtained as the mechanical signals of the user using the pressure sensor. In some embodiments, the mechanical signals are obtained based on the posture signals and the EMG signals. In some embodiments, the EMG signals, the ECG signals, the posture signals, the respiratory signals, the sweat signals, etc., or any combination thereof, are used to characterize the physiological state (e.g., a muscle efficiency, a muscle fatigue state, an injury risk for the motion, etc.) of the user during running.

In some embodiments, the processing device 110 obtains the motion data directly from the data collection device (e.g., the data collection device 150). In some embodiments, the motion data is stored in a storage device (e.g., the storage device 130), and the processing device 110 obtains the motion data from the storage device.

In some embodiments, a plurality of sets of motion data of the user at a plurality of preset stride frequencies are obtained. Each of the plurality of preset stride frequencies is located in a specific frequency range (also referred to as a step range), and each of the plurality of sets of motion data corresponds to one of the plurality of preset stride frequencies. In some embodiments, the stride frequency is a count of steps of running per unit time (e.g., per minute). Merely by way of example, a professional runner has a stride frequency of 180 steps per minute. In some embodiments, each of the preset stride frequencies is a preset stride frequency within a specific frequency range. The plurality of preset stride frequencies correspond to different frequency ranges. In some embodiments, the frequency ranges of the preset stride frequencies are default. In some embodiments, the frequency ranges of the preset stride frequencies are personalized for different users. The set preset stride frequencies and/or the corresponding frequency ranges are stored in the storage device. In some embodiments, the plurality of preset stride frequencies include a first preset stride frequency, a second preset stride frequency, ...... an Nth preset stride frequency, which correspond to different frequency ranges. Merely by way of example, the first preset stride frequency is located at or corresponds to a frequency range of 100 steps per minute to 110 steps per minute, the second preset stride frequency is located at or corresponds to a frequency range of 110 steps to 120 steps per minute, ..., the Nth preset stride frequency is located at or corresponds to a frequency range of 180 steps per minute to 190 steps per minute. In some embodiments, the motion data at each of the plurality of preset stride frequencies is obtained respectively.

In some embodiments, the plurality of sets of motion data at the plurality of preset stride frequencies are obtained during the running of the user. For example, when the user is running at the first preset stride frequency, the first set of motion data of the user at the first preset stride frequency is obtained; then, the user adjusts the stride frequency to the second preset stride frequency, and the second set of motion data of the user at the second preset stride frequency is obtained, ..., and similarly, an Nth motion data of the user at an Nth preset stride frequency is obtained. In some embodiments, the plurality of sets of motion data at different preset stride frequencies are obtained based on a historical motion record (e.g., a stride frequency record, a motion data record corresponding to the stride frequency record) of the user. In some embodiments, each of the plurality of sets of motion data characterizes the physiological state of the user at the corresponding preset stride frequency.

In some embodiments, the preset stride frequency is fed back to the user via a feedback manner (e.g., a voice manner, a vibration manner, etc.) so that the user follows the preset stride frequency to move to generate the motion data at the preset stride frequency. For example, the preset stride frequency is fed back to the user through the voice manner, and the wearable device or the terminal device (e.g., a cell phone) plays a voice rhythm. A frequency of the voice rhythm is the same as the preset stride frequency, the user runs following the voice rhythm, and the data collection device is utilized to obtain the motion data of the user during the running process. The motion data is the motion data generated at the preset stride frequency.

In some embodiments, the obtaining a plurality of sets of motion data of the user at the plurality of preset stride frequencies includes: obtaining physiological information of the user, and determining the plurality of sets of motion data of the user at the plurality of preset stride frequencies based on the physiological information. In some embodiments, the physiological information of the user includes body information and motion information. The body information refers to information related to the physical condition or a physical feature of the user. In some embodiments, the body information includes, but is not limited to, one or more of a gender, age, height, weight, body composition information, etc. Merely by way of example, the body composition information includes a body fat percentage, a muscle mass, a skeletal muscle percentage, a bone mass, subcutaneous fat, body water, etc. The motion information refers to information related to a historical motion of the user. The motion information includes a motion habit, a motion performance, etc. Merely by way of example, the motion habit includes, but is not limited to, a motion frequency, a motion type, a motion duration, etc. The physiological information corresponds to different users is stored in the storage device. In some embodiments, the physiological information of different users (e.g., as testers used to obtain the plurality of sets of motion data) is pre-collected and stored in the storage device. When obtaining the plurality of sets of motion data of the user at the plurality of preset stride frequencies, the physiological information of the user is obtained from the storage device, and based on the physiological information of the user, the plurality of sets of motion data under the plurality of preset stride frequencies are determined. For example, the testers include a user 1, a user 2, ..., a user m, and the physiological information of the m users are stored in the storage device. When obtaining the plurality of sets of motion data of the user 1 at the plurality of preset stride frequencies, the physiological data of the user 1 is obtained from the storage device, and based on the physiological data of the user 1, the plurality of sets of motion data (e.g., the EMG signals, the posture signals, the ECG signals, etc.) of the user 1 at the different preset stride frequencies are determined. Similarly, the plurality of sets of motion data of different users (i.e., the users with different physiological information) at different preset stride frequencies are determined respectively. In some embodiments, the motion data of the users with different physiological information is different at the same preset stride frequency, and the physiological information of the users affects the motion data generated by the users during motion, thereby affecting the physiological state of the user. In some embodiments, a relationship between the stride frequencies and the physiological states of the user under different conditions (e.g., different physiological information) is determined based on the physiological information of the user, as described elsewhere in the present disclosure (e.g., operation 330).

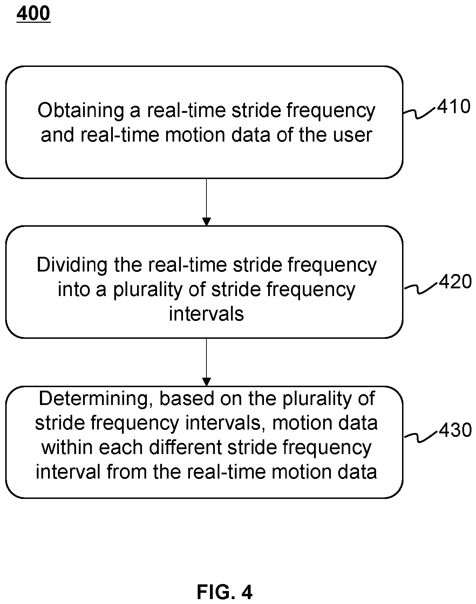

In some embodiments, the motion data of the user at different stride frequencies is obtained in other manners. For example, real-time motion data of the user is obtained based on a real-time stride frequency of the user, as described in FIG. 4 and the related descriptions thereof.

In some embodiments, a motion speed of the user is correlated with the stride frequency, and the stride frequency is taken as an indicator for evaluating the motion speed. Generally, the motion speed is approximated as a product of the stride frequency and a stride length. In such cases, when the motion speed of the user remains stable (i.e., a uniform motion or a near-uniform motion) or is located in a specific range interval, the motion data of the user obtained at a current speed or a speed range is used as a stable criterion for determining the stride frequency. In some embodiments, the obtaining the motion data of the user at different stride frequencies includes: determining a target speed interval; and obtaining the motion data at different stride frequencies within the target speed interval based on the target speed interval.

The speed interval refers to a range of values of a running speed during the motion of the user. The target speed interval is a preset speed interval with a specific speed range. In some embodiments, the speed range defined by the target speed interval is relatively small, i.e., there is less variation in the running speed within the target speed interval. When the motion speed of the user is within the target speed interval, the motion data obtained based on the target speed interval is used as a stable criterion to determine the stride frequency. Merely by way of example, when the user moves at the first preset stride frequency within the target speed interval, the motion data obtained at this time is the first motion data; when the user moves at the second preset stride frequency within the target speed interval, the motion data obtained at this time is the second motion data; ...; and when the user moves at the Nth preset stride frequency within the target speed interval, the motion data obtained at this time is the Nth motion data. An influencing factor of a difference between the first motion data, the second motion data, ..., and the Nth motion data is mainly the stride frequency, and the motion speed is ignored. In some embodiments, when the motion speed of the user is located in the target speed interval and the user is moving at the preset stride frequency, the motion data obtained by the data collection device is valid, and the motion data corresponds to the preset stride frequency and is used for subsequent analytical processing (e.g., determining the physiological state of the user). When the motion speed of the user exceeds the target speed interval, regardless of whether or not the user is moving at the preset stride frequency, the data collection device does not collect the motion data, or the collected motion data is invalid, and the invalid motion data is not used for the subsequent analytical processing. In some embodiments, a suitable target speed interval is set according to an actual situation (e.g., different users, road environments) as well as an application scenario, and the present disclosure does not make any specific limitations on a value range of the target speed interval.

By reasonably setting the range of the target speed interval, the influence of different motion speeds at different stride frequencies on the obtained motion data is reduced, thus ensuring the accuracy of the motion data.

In some embodiments, to reduce the difference in motion data due to different motion speeds at different stride frequencies, the motion data of the user at different stride frequencies but at the same motion speed is obtained. In some embodiments, the motion data of the user at different stride frequencies and different motion speeds is obtained. In this situation, the motion speed of the user is monitored, and the influence brought by different motion speeds is compensated in a result of the motion data.

In 320, the physiological state of the user is determined based on the motion data.

In some embodiments, operation 320 is performed by the processing module 220. In some embodiments, the physiological state is used to characterize the physiological situation of the user and a state of change thereof during the running. In some embodiments, the physiological state includes the muscle efficiency, a muscle fatigue (also referred to as the muscle fatigue state), and a muscle tension. In some embodiments, when the user runs at different stride frequencies, the muscle efficiency, the muscle fatigue state, and the muscle tension change may be different, which results in different motion efficiencies (e.g., running performances, fitness results, etc.) of the user. In some embodiments, the physiological state of the user is determined based on the EMG signals. For example, the physiological state of the user is determined based on the feature values of the EMG signals. The feature values of the EMG signals are characteristic parameters of the EMG signals that reflect the physiological state of the user. Exemplary characteristic parameters include amplitudes, an absolute value of the amplitudes, an average value of the amplitudes (or an absolute value), a maximum value of the amplitudes (or an absolute value), a minimum value of the amplitudes (or an absolute value), a median frequency, a peak frequency, a total value of the force, an EMG power, a foot touch time, an average frequency, etc. of the EMG signals.

In some embodiments, whether the muscles of the user are in the fatigue state is determined based on the EMG signals in the motion data. For example, a total value of force generated by leg muscles in the fatigue state is greater than a total value of force generated by the leg muscles in a non-fatigue state. The total value of force is characterized by a sum of the amplitudes (e.g., a sum of the absolute amplitudes, a sum of the multiple squares of amplitudes, a sum of the multiple amplitudes, etc.) of the EMG signals over a preset time period (e.g., a running cycle, a time period in which the foot touches the ground, a time period in which the foot is in the air, etc.). With the sum of the amplitudes of the EMG signals as the characteristic parameter, whether the muscles of the user are in the fatigue state is determined. As another example, the EMG power of the leg muscles in the fatigue state is greater than the EMG power of the leg muscles in the non-fatigue state. The EMG power refers to the sum of amplitudes of the EMG signals per unit time. With the EMG power of the EMG signals as the characteristic parameter, whether the muscles of the user are in the fatigue state is determined. As another example, in the non-fatigue state, a portion of the leg muscles are relaxed when the leg is in the air, and the corresponding amplitude of the EMG signals is the minimum value in a cycle; and when entering the fatigue state, the portion of the leg muscles is in a tightened state when the leg is in the air, which is manifested, for example, as an increase in the amplitude of the EMG signals of the muscles when the leg is in the air. With the amplitude of the EMG signals of the leg muscles when the leg is in the air as the characteristic parameter, whether the muscles of the user are in the fatigue state is determined. As another example, in the non-fatigue state, a portion of the leg muscles are tightened when the foot touches the ground, and the amplitude of the corresponding EMG signals is the maximum value in a cycle; and when entering the fatigue state, muscle strength of the portion of the leg muscles increases abnormally when foot touches the ground, which is manifested, for example, as the amplitude of the EMG signals increases at a speed greater than a speed threshold. With the increased speed of the amplitude of the EMG signals of the leg muscles when the foot touches the ground as the characteristic parameter, whether or not the muscle of the user is in the fatigue state is determined. As another example, when the leg muscles enter the fatigue state from the non-fatigue state, the average frequency, the median frequency, etc. of the leg muscles drop. The average frequency is a ratio of a weighted sum of the amplitudes of the EMG signals at each frequency (e.g., the amplitude at each frequency multiplied by the corresponding frequency, multiple squares of the amplitude at each frequency, multiple values of the amplitude at each frequency, etc.) to a sum of the amplitudes. With the average frequency of the EMG signals as the characteristic parameters, whether the muscles of the user are in a fatigue state is determined. As another example, the muscle strength of the leg muscles in the fatigue state is greater than the muscle strength in the non-fatigue state when performing the same running action. And as the degree of fatigue increases, the muscle strength further increases. When the muscle strength is too high, the leg muscles may be damaged due to excessive fatigue. Thus, the fatigue state of the muscles is determined based on an intensity change of the EMG signals. Specifically, the EMG signals are segmented according to a plurality of time windows divided by time periods, and an average of the amplitudes (e.g., the absolute values of the amplitudes) of the EMG signals of each segment is used as the feature value of the EMG signals of each segment, thereby determining a plurality of feature values within the plurality of time periods. The average of the amplitudes reflects the average intensity of each segment of the EMG signals in each time period, such that the change in the average intensity of the EMG signals during running is determined based on the plurality of feature values, and the fatigue state of the muscles may be characterized.

As the running time increases, the leg muscles gradually enter the fatigue state, and the muscle efficiency gradually decreases. The muscle efficiency refers to the muscle output required to maintain the same motion capacity. In some embodiments, the higher the muscle output required to maintain the same motion capacity, the lower the muscle efficiency. In some embodiments, the muscle efficiency is determined based on an integral value of the EMG signals in a specific interval (e.g., a specific time period). In some embodiments, the muscle efficiency is also determined based on the average of the amplitudes of the EMG signals in a specific interval.

In some embodiments, the level of the muscle tension of the user is determined based on the EMG signals. The level of the muscle tension is the degree to which the muscles relax during motion. During the running of the user, the leg muscles move periodically. When the leg is in the air, a portion of the leg muscles (e.g., calf muscles) are relaxed, and the amplitude of the corresponding EMG signals is the minimum value within a cycle; and when the leg (i.e., the foot) touches the ground, a portion of the leg muscles are tightened, and the amplitude of the corresponding EMG signals is a maximum value within the cycle. In such cases, the force generation of the leg muscles are determined using the amplitude (e.g., the minimum value, the maximum value) of the EMG signals as the characteristic parameter, thereby determining the tension state of the leg muscles. For example, the EMG signals are segmented according to a plurality of time windows divided by time periods, and the minimum value of the amplitudes (e.g., the absolute value of the amplitudes) of the EMG signals in each segment is used as the feature value of the EMG signals in each segment, thereby determining a plurality of feature values within the plurality of time periods. The minimum value of the amplitudes reflects a minimum intensity of each segment of the EMG signals in each time period, so that changes of the minimum intensity of the EMG signals during running are determined based on the plurality of feature values, and the level of the muscle tension is characterized.

In 330, a recommended stride frequency for the user is determined based on the physiological state.

In some embodiments, operation 330 is performed by the recommendation module 230. In some embodiments, the fatigue state, the muscle efficiency, and the muscle tension of the muscles of the user when the user is running at different stride frequencies are different, which makes the motion efficiency of the user different. A correspondence between the physiological states and the stride frequencies of the user is determined based on at least one of the muscle efficiency, the muscle tension, and the fatigue state, and based on the correspondence, the recommended stride frequency for the user is determined. When running at the recommended stride frequency, the motion efficiency of the user is improved (e.g., so that the muscle efficiency is in an optimal state, the fatigue state and the level of the muscle tension is within a target interval), and the injury risk for the motion of the user can be prevented, which ensures the scientificity and safety of motion.

In some embodiments, a correspondence between physiological states and the stride frequencies, which is determined based on the muscle fatigue, the muscle efficiency, and/or the muscle tension, is referred to as a first correspondence. In some embodiments, the first correspondence includes a correspondence between muscle efficiencies and the stride frequencies. In some embodiments, a correspondence between the muscle efficiencies and a plurality of preset stride frequencies is determined. In some embodiments, the EMG signals at the first preset stride frequency are analyzed and processed (e.g., a feature value calculation processing) to obtain the muscle efficiency at the first preset stride frequency (referred to as a first muscle efficiency), thereby determining a correspondence between the first preset stride frequency and the first muscle efficiency. That is, the first muscle efficiency corresponds to the first preset stride frequency. Similarly, the correspondence between the second preset stride frequency and the second muscle efficiency, the correspondence between the third preset stride frequency and the third muscle efficiency,..., the correspondence between the Nth preset stride frequency and the Nth muscle efficiency are determined. In some embodiments, the correspondence between the muscle efficiencies and the real-time stride frequencies is determined. In some embodiments, a real-time stride frequency of the user and real-time EMG signals corresponding to the real-time stride frequency are obtained, and the real-time EMG signals are analyzed and processed to obtain a real-time muscle efficiency at the real-time stride frequency.

In some embodiments, the recommended stride frequency for the user is determined based on the correspondence between the muscle efficiencies and the stride frequencies. In some embodiments, based on the correspondence between the muscle efficiencies and the stride frequencies, one or more stride frequencies are determined from different stride frequencies as the recommended stride frequencies. For example, one or more of the different stride frequencies (e.g., the plurality of preset stride frequencies) corresponding to a relatively high muscle efficiency (or a relatively low muscle fatigue state) are determined as the recommended stride frequencies.

In some embodiments, the first correspondence includes a correspondence between the muscle tensions and the stride frequencies. In some embodiments, the correspondences between the muscle tensions and a plurality of preset stride frequencies are determined. Merely by way of example, the EMG signals at the first preset stride frequency are analyzed and processed to obtain the muscle tension at the first preset stride frequency (referred to as a first muscle tension), so as to determine the correspondence between the first preset stride frequency and the first muscle tension. That is, the first muscle tension corresponds to the first preset stride frequency. Similarly, a correspondence between the second preset stride frequency and the second muscle tension, a correspondence between the third preset stride frequency and the third muscle tension,..., a correspondence between the Nth preset stride frequency and the Nth muscle tension are determined. In some embodiments, the correspondence between the muscle tensions and real-time stride frequencies is determined. In some embodiments, the real-time stride frequency of the user and the real-time EMG signals corresponding to the real-time stride frequency are obtained, and the real-time EMG signals are analyzed and processed to obtain a real-time muscle tension at the real-time stride frequency.

In some embodiments, the recommended stride frequency for the user is determined based on the correspondence between the muscle tensions and the stride frequencies. In some embodiments, based on the correspondence between the muscle tensions and the stride frequencies, one or more stride frequencies are determined from different stride frequencies as the recommended stride frequencies. For example, one or more of the different stride frequencies (e.g., the plurality of preset stride frequencies) corresponding to the muscle tension within a tolerance range of a human body and with a high degree are determined as the recommended stride frequencies.

In some embodiments, the first correspondence includes a correspondence between the muscle fatigues and the stride frequencies. In some embodiments, the correspondence between the muscle fatigues and the plurality of preset stride frequencies are determined. Merely by way of example, the EMG signals at the first preset stride frequency are analyzed and processed to obtain the muscle fatigue at the first preset stride frequency (referred to as a first muscle fatigue), so as to determine the correspondence between the first preset stride frequency and the first muscle fatigue. That is, the first muscle fatigue corresponds to the first preset stride frequency. Similarly, a correspondence between the second preset stride frequency and a second muscle fatigue,... a correspondence between the Nth preset stride frequency and a Nth muscle fatigue are determined. In some embodiments, a correspondence between the muscle fatigues and the real-time stride frequencies is also determined. In some embodiments, the real-time stride frequency of the user and the real-time EMG signals corresponding to the real-time stride frequency are obtained, and the real-time EMG signals are analyzed and processed to obtain the real-time muscle fatigue at the real-time stride frequency.

In some embodiments, the recommended stride frequency for the user is determined based on the correspondence between the muscle fatigues and the stride frequencies. In some embodiments, based on the correspondence between the muscle fatigues and the stride frequencies, one or more stride frequencies are determined from different stride frequencies as the recommended stride frequencies. In some embodiments, the correspondence between the muscle efficiencies and the stride frequencies, the correspondence between the muscle tensions and the stride frequencies, and the correspondence between the muscle fatigues and the stride frequencies are used together to determine the recommended stride frequency for the user.

In some embodiments, the correspondence between the stride frequencies and the physiological states of the user is determined based on a correspondence determination model. In some embodiments, inputs to the correspondence determination model include, but are not limited to, the stride frequencies. An output of the correspondence determination model is the correspondence between the stride frequencies and the physiological states (e.g., the muscle fatigue, the muscle tension, the muscle efficiency, the injury risk for the motion, etc.). In some embodiments, the correspondence determination model is a trained machine learning model. In some embodiments, the correspondence determination model is pre-trained by the processing device and stored in the storage device, and the processing device accesses the storage device to obtain the correspondence determination model. In some embodiments, the machine learning model includes one or more of a linear classification model (LR), a support vector machine model (SVM), a naive Bayesian model (NB), a k-nearest neighbor model (KNN), a decision tree model (DT), an integration model (RF/GDBT, etc.), etc.

In some embodiments, the correspondence determination model is obtained through a training process performed based on sample information. The sample information includes different preset stride frequencies of different users. In some embodiments, when the stride frequency is used as the sample information, the physiological state of the user is monitored while the stride frequency is obtained. In some embodiments, the sample information is labeled when training the machine learning model. For example, the body of the user has a first physiological state when moving at the first preset stride frequency, and the first preset stride frequency is labeled as "the first preset stride frequency corresponding to the first physiological state"; the body of the user has a second physiological state when moving at the second preset frequency, and the second preset stride frequency is labeled as "the second preset stride frequency corresponds to the second physiological state". Different preset stride frequencies correspond to different physiological states. The correspondence determination model is trained using the labeled sample information (i.e., each labeled preset stride frequency) as the input to the machine learning model. The correspondence between the stride frequency and the physiological state is output when the stride frequency (which includes the real-time stride frequency) is input to the correspondence determination model.

In some embodiments, to improve the accuracy of the correspondence between the preset stride frequencies and the physiological state, the physiological information of the user is also used as the input of the machine learning model for training. As a result, the correspondence between the stride frequencies and the physiological state under different conditions (e.g., different physiological information) is obtained. Further, the recommended stride frequency for the user is determined based on the correspondence, thereby making the recommended stride frequency more suitable for the user. In some embodiments, the recommended stride frequency under a target training task (also referred to as an initial recommended stride frequency) is estimated based on the actual input information of the user. The user first moves at the initial recommended stride frequency, the real-time motion data of the user is collected during the motion process, and the system for processing motion data iteratively updates the correspondence determination model based on the real-time motion data, so as to make the correspondence determination model output a correspondence more suitable for actual needs. Based on the correspondence, a recommended stride frequency that more accurately fits the real-time motion state of the user (i.e., an iteratively updated recommended stride frequency) is re-determined. In some embodiments, the system for processing motion data collects more motion data accumulated during a long-term motion, so as to make the recommended stride frequency recommended by the system for processing motion data more reasonable and more closely fits the motion habit of the user.

In some embodiments, the physiological state and the recommended stride frequency of the user are also determined in other ways or manners. For example, the physiological state of the user and the recommended stride frequency are determined based on the ECG signals and/or the posture signals, details of which can be found in FIG.5 and the related descriptions thereof. In some embodiments, the recommended stride frequency for the user is determined based on one or any combination of the correspondence between the muscle efficiencies and the stride frequencies, the correspondence between the muscle tensions and the stride frequencies, and the correspondence between other physiological states (e.g., the injury risk for the motion) and stride frequencies. In such cases, the correspondence between the physiological state determined based on a variety of motion data such as the EMG signals, the posture signals, the ECG signals, the respiratory signals, etc., and the stride frequencies is considered comprehensively to determine the recommended stride frequency.

In some embodiments, the recommended stride frequency is determined based on the historical motion of the user. In some embodiments, the recommended stride frequency is determined based on historical EMG signals. For example, the recommended stride frequency for a current user is determined based on a historical stride frequency corresponding to the muscle fatigue determined based on the historical EMG signals. Merely by way of example, the recommended stride frequency is less or slightly less than the historical stride frequency. As another example, a historical recommended stride frequency is combined with current EMG signals to determine the recommended stride frequency for the current user.

In some embodiments, the recommended stride frequency is determined in conjunction with road environment (e.g., an uphill, a downhill) as well as the personal physical condition (e.g., a historical motion record) of the user. For example, when the user is running on an inclined section of the road or on a treadmill, the recommended stride frequency for the inclined section is appropriately smaller than the recommended stride frequency for a gentle section under the same physiological state, so as to prevent a knee injury. In some embodiments, when the system does not record the real-time physical state of the user or a situation in which the user is located (e.g., the road environment), an initial stride frequency is first recommended to the user (determined based on a historical motion record of the user or determined based on running data of other groups of people). After the user moves for a certain period of time under the initial stride frequency, the physiological state of the user is determined based on the motion data during the period of time, and the recommended stride frequency is then determined according to the physiological state.

In 340, the recommended stride frequency is fed back to the user.

In some embodiments, operation 340 is performed by the feedback module 240. In some embodiments, after the recommended stride frequency for the user is determined based on the aforementioned operations 310, 320, and 330, the recommended stride frequency is further fed back to the user. The user may adjust his or her stride frequency according to the recommended stride frequency, thereby improving the motion efficiency.

In some embodiments, a beat that is the same as the recommended stride frequency is fed back to the user based on a preset feedback manner. The user adjusts his/her stride frequency based on the recommended stride frequency so that the stride frequency is the same as the recommended stride frequency. Then the user can run at the recommended stride frequency. In some embodiments, the preset feedback manner includes at least one of voice feedback, vibration feedback, light feedback, and display feedback. In some embodiments, the beat that is the same as the recommended stride frequency is fed to the user based on a voice feedback manner. For example, a device (e.g., a cell phone, a watch, a headset) with a sound output function in the system for processing motion data plays a voice beat that is the same as the recommended stride frequency, and the user adjusts his or her stride frequency according to the voice beat to make the stride frequency same or substantially same as the recommended stride frequency. In some embodiments, the beat that is the same as the recommended stride frequency is fed back to the user based on a vibration feedback manner. For example, the device (e.g., the cell phone, the watch) with a vibration generation function in the system for processing motion data may vibrate at the recommended stride frequency. In some embodiments, the beat that is the same as the recommended stride frequency is fed to the user based on a light feedback manner. For example, an electronic device (e.g., the cell phone, the watch) in the system for processing motion data performs a light flashing with the same frequency as the recommended stride frequency. In some embodiments, the beat that is the same as the recommended stride frequency is fed back to the user based on a display feedback manner. For example, the electronic device (e.g., the cell phone) in the system for processing motion data may display the recommended stride frequency.

In some embodiments, the recommended stride frequency is fed back to the user via a virtual reality device and/or an augmented reality device (e.g., AR glasses). For example, the user wears the AR glasses during running, the recommended stride frequency is displayed in the form of data in the AR glasses, and the user adjusts his or her stride frequency according to the data of the recommended stride frequency. By using the AR glasses to feed back the recommended stride frequency to the user, the user ca be more clearly informed of the recommended stride frequency, and the reliance of the user on the cell phone, the watch, and a bracelet can be reduced, thereby reducing a burden of the user during running.

In some embodiments, the recommended stride frequency is also fed back to the user based on a biofeedback manner (e.g., an electrical stimulation). In some embodiments, stimulation is performed to a body portion of the user based on the biofeedback manner to provide feedback to the user on the recommended stride frequency. The stimulation performed to the body portion of the user has a stimulation frequency that is the same as the recommended stride frequency. In some embodiments, electrodes are provided on a wearing body (e.g., a top, pants, a belt, etc.) of the wearable device, and the electrodes are in contact with human skin when the user is wearing the wearable device. The processing device controls the electrodes to stimulate the body portions of the user based on the stimulation frequency (i.e., the recommended stride frequency).

The user adjusts his/her real-time stride frequency according to the recommended stride frequency, so that the real-time stride frequency is consistent with the recommended stride frequency and the user runs under the recommended stride frequency. As a result, the user can move more scientifically and efficiently, thereby improving motion efficiency.

It should be noted that the descriptions of process 300 are intended to be exemplary and illustrative only and do not limit the scope of application of the present disclosure. For those skilled in the art, various corrections and changes are made to the process 300 under the guidance of the present disclosure. For example, the motion data of the user is not limited to the EMG signals, the posture signals, and the ECG signals described above, but can also be other physiological parameter signals, such as temperature signals, humidity signals, a blood oxygen concentration, a respiratory frequency, etc., and all the physiological parameter signals involved in human motion are considered as the motion data in the embodiment of the present disclosure. As another example, operation 340 can be omitted. However, these corrections and changes remain within the scope of the present disclosure.

FIG. 4 is a flowchart illustrating an exemplary process for obtaining motion data according to some embodiments of the present disclosure. In some embodiments, a process 400 is performed by the obtaining module 210. The process 400 includes the following operations.

In 410, a real-time stride frequency and real-time motion data of the user are obtained.

In some embodiments, when the user wears the wearable device for running, the obtaining module 210 obtains a real-time stride frequency as well as real-time motion data of the user. In some embodiments, the real-time stride frequency of the user changes during different time periods during the running. For example, the real-time stride frequency tends to gradually increase or decrease. Merely by way of example, the real-time stride frequency of the user is relatively high when the user starts running; and after running for a period of time, the real-time stride frequency gradually decreases. As another example, the real-time stride frequency of the user is sometimes high and sometimes low. As a result, the motion data generated by running at different real-time stride frequencies, that is, the real-time motion data corresponding to the real-time stride frequency, is also different, and the real-time stride frequency and the real-time motion data of the user are obtained.

In some embodiments, the real-time stride frequency is obtained by a data collection device. In some embodiments, the real-time stride frequency is obtained via an inertial sensor. Merely by way of example, a running motion is a periodic motion. The running motion consists of a plurality of running motions repeated periodically, and signals collected by the inertial sensor (e.g., the posture sensor) are correspondingly periodic signals (or approximately periodic signals). A completion of one running motion by the user is regarded as a completion of a periodic motion, in this way, the real-time stride frequency is obtained based on the inertial sensor. In some embodiments, the real-time stride frequency is extracted from EMG signals. For example, when the user is running, the periodic running motion generates periodically changing EMG signals. By extracting a period of the EMG signals, the real-time stride frequency is obtained.

In 420, the real-time stride frequency is divided into a plurality of stride frequency intervals.

In some embodiments, the real-time stride frequency is divided into the plurality of stride frequency intervals based on a plurality of stride frequency ranges, with each stride frequency interval corresponding to a stride frequency range. For example, the plurality of stride frequency ranges include 150 steps/min-160 steps/min, 160 steps/min-170 steps/min, 170 steps/min-180 steps/min, 180 steps/min- 190 steps/minute, 200 steps/minute-210 steps/minute, and the real-time stride frequency is divided into 5 corresponding stride frequency intervals according to the stride frequency ranges. Each stride frequency interval has a corresponding stride frequency. That is, the stride frequency of a first stride frequency interval is 140 steps/minute-150 steps/minute,..., and the stride frequency of a fifth stride frequency interval is 200 steps/minute-210 steps/minute. In some embodiments, the stride frequency range is relatively small to ensure that a difference of real-time motion data within a single stride frequency interval is relatively small, thereby ensuring the accuracy of the motion data at different stride frequencies. The stride frequency range is set to be smaller. In some embodiments, the difference between upper and lower limits of the stride frequency range is less than 10 (in steps/minute). In some embodiments, the difference between the upper and the lower limits of a preset range is set less than 8. In some embodiments, the difference between the upper and the lower limits of the preset range is less than 6. In some embodiments, the difference between the upper and the lower limits of the preset range is less than 5. In some embodiments, the difference between the upper and the lower limits of the preset range is less than 3. In some embodiments, the preset range is set according to actual needs, which is not limited in the embodiments of the present disclosure.

In 430, the motion data within each different stride frequency interval is determined from the real-time motion data based on the plurality of stride frequency intervals.

In some embodiments, the real-time motion data is different at different real-time stride frequencies. For example, when a change in the real-time stride frequencies is great, a change in the real-time motion data is also great; and when the change in the real-time stride frequencies is small, the change in the real-time motion data is also small. In some embodiments, the motion data corresponding to the stride frequency interval is determined based on different stride frequency intervals. For example, at least a portion of the real-time motion data corresponding to the same stride frequency interval is concentrated as the motion data corresponding to the stride frequency interval. Merely by way of example, a motion speed corresponding to the real-time stride frequency is or is not within a target speed interval (i.e., the motion speed of the user is or is not within the target speed interval when the data collection device collects the real-time stride frequency). When the motion speed corresponding to the real-time stride frequency lies in the target speed interval, all of the real-time motion data corresponding to the same stride frequency interval is concentrated together as the motion data corresponding to the stride frequency interval. When the motion speed corresponding to the real-time stride frequency is partially located in the target speed interval, the real-time stride frequency corresponding to the motion speed within the target speed interval is filtered out, and then the real-time motion data corresponding to the real-time stride frequency which are filtered out and located in the same stride frequency interval is concentrated together as the motion data corresponding to that stride frequency interval.

In some embodiments, a manner of obtaining the motion data based on the real-time stride frequency has a higher flexibility compared to obtaining the motion data based on a preset stride frequency. This is due to the fact that when obtaining the motion data based on the preset stride frequency, the user needs to keep the stride frequency within a preset stride frequency interval, and if the stride frequency of the user exceeds the preset stride frequency interval, the accuracy of the motion data is affected. Obtaining the motion data based on the real-time stride frequency does not require controlling the stride frequency of the user. The user can move freely with different stride frequencies.

It should be noted that the descriptions of the process 400 are intended to be exemplary and illustrative only and do not limit the scope of application of the present disclosure. For those skilled in the art, various corrections and changes are made to the process 400 under the guidance of the present disclosure. However, these corrections and changes remain within the scope of the present disclosure.

FIG. 5 is a flowchart illustrating an exemplary process for determining a recommended stride frequency according to some embodiments of the present disclosure. In some embodiments, a process 500 is performed by the determination module 220. Process 500 includes the following operations.

In 510, a correspondence between physiological states of the user and stride frequencies is determined based on ECG signals and/or posture signals.

In some embodiments, the physiological state also includes an injury risk for the motion. Incorrect motions of the user during running, e.g., an incorrect running posture, an inappropriate running stride frequency, a fast heart rate, etc., may lead to damage on the human body as well as failure to achieve an expected motion efficiency. In some embodiments, the injury risk for the motion includes an injury risk for a posture. The injury risk for a posture includes an injury error, a compensation error, an efficiency error, a symmetry error, etc., or any combination thereof. The injury error refers to that the motion error causes an injury to the human body. The compensation error refers to an error that uses a non-target portion (e.g., knees, ankles) to assist in exerting force. The efficiency error refers to that when performing a running motion, a range of swings or steps is too great or too small such that a target portion is in a non-optimal level of activation. The symmetry error refers to a situation where two symmetrical (e.g., bilateral symmetrical, anterior-posterior symmetrical) portions of the human body are not balanced in exerting force. In some embodiments, the injury risk for the motion includes injury scales. Merely by way of example, the injury risk scales include a severe, a moderate, a mild, etc.

In some embodiments, the injury risk for the motion of the user is determined based on the posture signals. The posture signals include information such as angles, speeds, and accelerations of various joints, or Euler angles, angular speeds, and angular accelerations of various body portions. In some embodiments, the posture signals are used to characterize the technical accuracy of the current motion of the user (e.g., the joint angles, a force sequence, etc.), thereby determining the injury risk for the motion (e.g., a knee injury, an ankle injury). In some embodiments, the posture signals are obtained by a posture data collection device. Exemplary posture data collection devices include a velocity sensor, an inertial sensor (e.g., an acceleration sensor, an angular velocity sensor (e.g., a gyroscope), etc.), an optical sensor (e.g., an optical distance sensor, a video/image grabber), an acoustic distance sensor, a tension sensor, etc., or any combination thereof.

In some embodiments, the injury risk for the motion includes an injury risk for the heart. As the stride frequency increases, the heart rate gradually accelerates, and the injury risk for the heart occurs when the heart rate is larger than a heart rate threshold. In some embodiments, the injury risk for the heart of the user is determined based on the ECG signals. The ECG signals refer to signals that indicate the heart activity of the user. In some embodiments, the ECG signals are collected by an ECG data collection device. For example, the ECG data collection device includes a plurality of electrodes used to fit different portions of the user for collecting the ECG signals of the user.

In some embodiments, a correspondence between the physiological states and the stride frequencies determined based on the ECG signals and/or the posture signals is also referred to as a second correspondence. The second correspondence includes a correspondence between the injury risks for the posture and the stride frequencies. In some embodiments, the correspondence between the injury risks for the posture and a plurality of preset stride frequencies is determined. In some embodiments, the posture signals at a first preset stride frequency are processed (e.g., feature value processing) to obtain the injury risk for the posture at the first preset stride frequency (referred to as a first injury risk for the posture), thereby determining a correspondence between the first preset stride frequency and the first injury risk for the posture. That is, the first injury risk for the posture corresponds to the first preset stride frequency. Similarly, the correspondence between a second preset stride frequency and a second injury risk for the posture, a correspondence between a third preset stride frequency and a third injury risk for the posture,..., and a correspondence between a Nth preset stride frequency and a Nth injury risk for the posture are determined. In some embodiments, the correspondence between the injury risk for the posture and a real-time stride frequency is also determined.

In some embodiments, the second correspondence includes a correspondence between the injury risks for the heart and the stride frequencies. In some embodiments, the correspondence between the injury risks for the heart and the stride frequencies of the user is determined based on the ECG signals. In some embodiments, the correspondence between the injury risks for the heart and the plurality of preset stride frequencies is determined. In some embodiments, the ECG signals at the first preset stride frequency are processed to obtain the injury risk for the heat at the first preset stride frequency (referred to as the first injury risk for the heat), i.e., the first injury risk for the heart corresponds to the first preset stride frequency. Similarly, the correspondence between a second preset stride frequency and a second injury risk for the heart, a third preset stride frequency and a third injury risk for the heart,..., and the correspondence between the Nth preset stride frequency and a Nth injury risk for the heart are determined. In some embodiments, the correspondence between the injury risk for the heart and the real-time stride frequency is also determined.

In 520, the recommended stride frequency for the user is determined based on the correspondence.

In some embodiments, the recommended stride frequency for the user is determined based on the correspondence between the injury risks for the motion and the stride frequencies. For example, the stride frequency with the lowest injury risk for the motion is determined as the recommended stride frequency. In some embodiments, one stride frequency is determined from different stride frequencies as the recommended stride frequency based on the correspondence between the injury risks for the motion and the stride frequencies. In some embodiments, one stride frequency is determined from different stride frequencies as the recommended stride frequency based on the correspondence between the injury risks for the heart and the stride frequencies.

It should be noted that, in other embodiments, the recommended stride frequency for the user is also determined in conjunction with the correspondence between the physiological states determined by the other motion data, such as respiratory signals, pressure signals, sweat signals, etc., and the stride frequency.

In some embodiments, the user runs with a certain respiratory rhythm, e.g., breathing once for a few steps. The respiratory rhythm of the user affects the physiological state of the user. In some embodiments, a strain sensor (or a contact impedance) is disposed on a waistband of the wearable device, and when the user breathes, the dimensions of the waistband change, and a signal value collected by the strain sensor changes. In some embodiments, the respiratory frequency of the user while running is detected by the strain sensor, and the respiratory rhythm is adjusted according to the respiratory frequency, which helps the user to perform motions more scientifically.

It should be noted that the descriptions of process 500 are intended to be exemplary and illustrative only and do not limit the scope of application of the present disclosure. For those skilled in the art, various corrections and changes are made to the process 500 under the guidance of the present disclosure. However, these corrections and changes remain within the scope of the present disclosure.

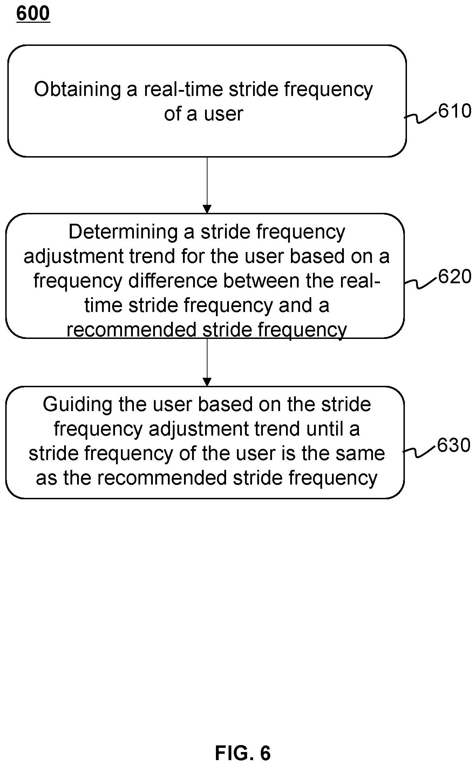

FIG. 6 is a flowchart illustrating an exemplary process for feeding back a recommended stride frequency according to some embodiments of the present disclosure. In some embodiments, a process 600 is performed by the feedback module 240. Process 600 includes the following operations.

In 610, a real-time stride frequency of the user is obtained.

In some embodiments, the real-time stride frequency refers to a stride frequency at a current moment when the user runs. In some embodiments, the real-time stride frequency is obtained through a data collection device. In some embodiments, the real-time stride frequency is obtained through an inertial sensor. More descriptions regarding the manner in which the stride frequency is obtained using the data collection device may be found elsewhere in the present disclosure, e.g., in FIG. 4 and the related descriptions, which are not repeated here.

In 620, a stride frequency adjustment trend for the user is determined based on a frequency difference between the real-time stride frequency and the recommended stride frequency.

In some embodiments, there is a frequency difference between the real-time stride frequency of the user and the recommended stride frequency. For example, when the real-time stride frequency of the user is great and greater than the recommended stride frequency, the frequency difference between the real-time stride frequency and the recommended stride frequency is positive. As another example, if the real-time stride frequency of the user is small and less than the recommended stride frequency, the frequency difference between the real-time stride frequency and the recommended stride frequency is negative. In some embodiments, the stride frequency adjustment trend includes a frequency decrease trend and a frequency increase trend. When the frequency difference between the real-time stride frequency and the recommended stride frequency is positive, the stride frequency adjustment trend is the frequency decrease trend; and when the frequency difference between the real-time stride frequency and the recommended stride frequency is negative, the stride frequency adjustment trend of the user is the frequency increase trend. In some embodiments, the stride frequency adjustment trend includes a constant frequency, in which case the real-time stride frequency is the same as the recommended stride frequency.

In 630, the user is guided based on the stride frequency adjustment trend until a stride frequency of the user is the same as the recommended stride frequency.

In some embodiments, the user adjusts his or her real-time stride frequency based on the recommended stride frequency to make the real-time stride frequency same as the recommended stride frequency. In some embodiments, when the stride frequency adjustment trend is the frequency decrease trend, the user is guided to gradually reduce his or her real-time stride frequency until the real-time stride frequency is the same as the recommended stride frequency. When the stride frequency adjustment trend is the frequency increase trend, the user is guided to gradually increase his or her real-time stride frequency until the real-time stride frequency is the same as the recommended stride frequency. In some embodiments, when the stride frequency adjustment trend is the constant frequency, the real-time stride frequency is the same as the recommended stride frequency, and the user does not need to adjust the stride frequency.

In some embodiments, when guiding the user to adjust the stride frequency based on the stride frequency adjustment trend, a dynamic beat with gradually changing frequency is fed back to the user based on a preset feedback manner (e.g., a voice feedback, a vibration feedback, a light feedback, a display feedback, a biofeedback manner, etc.). The dynamic beat has the same frequency change trend as the stride frequency adjustment trend. For example, when the stride frequency adjustment trend is a frequency decrease trend, an initial frequency of the dynamic beat is the real-time stride frequency of the user, a target frequency of the dynamic beat is the recommended stride frequency, and the frequency of the dynamic beat gradually decreases from the initial frequency to the target frequency. As another example, when the stride frequency adjustment trend is the frequency increase trend, the initial frequency of the dynamic beat is the real-time stride frequency of the user, the target frequency of the dynamic beat is the recommended stride frequency, and the frequency of the dynamic beat gradually increases from the initial frequency to the target frequency. In some embodiments, a frequency adjustment process of the dynamic beat changes uniformly. In some embodiments, the frequency adjustment process of the dynamic beat changes non-uniformly.

The manner of guiding the user to adjust the stride frequency adjustment trend makes the process of adjusting the stride frequency more scientific and healthier.

It should be noted that the descriptions of the process 600 are intended to be exemplary and illustrative only and do not limit the scope of application of the present disclosure. For those skilled in the art, various corrections and changes are made to the process 600 under the guidance of the present disclosure. However, these corrections and changes remain within the scope of the present disclosure.

In some embodiments, the recommended stride frequency described in the embodiments of the present disclosure is adjusted in real-time based on the state of the user during running. In some embodiments, the user adjusts his or her stride frequency according to the recommended stride frequency, so that after the stride frequency is the same as the recommended stride frequency, the user keeps running at the recommended stride frequency. While running at the recommended stride frequency, the data collection device continues to collect the motion data of the user. When the user runs at the recommended stride frequency for a certain period of time, and the motion data collected by the data collection device indicates that the physiological state of the user can be further improved, the recommended stride frequency is adjusted according to the current physiological state of the user, or the recommended stride frequency is re-determined. For example, when the current physiological state of the user is good and there is still room for improvement, for example, when muscles are not in a fatigue state, and when the muscle efficiency can be further improved, which indicates that the stride frequency of the user can be improved on a basis of the recommended stride frequency, the recommended stride frequency can be re-determined or updated according to the current physiological state of the user. As another example, if the current physiological state of the user is poor, for example, a heart rate is too fast and the muscles are in the fatigue state for a long time, which indicates that the stride frequency of the user can be reduced based on the recommended stride frequency, the recommended stride frequency can be re-determined or updated according to the current physiological state of the user. Through a real-time adjustment of the recommended stride frequency, a motion efficiency of the user in a running motion process is further improved.

According to the above descriptions, the method for processing motion data described in FIG. 3-FIG. 6 is to first determine the physiological state of the user based on the motion data of the user at different stride frequencies, and then determine the recommended stride frequency of the user based on the physiological state. In some embodiments, to reduce the time for processing and calculating the motion data to determine the recommended stride frequency of the user more quickly, the operation of determining the physiological state can be omitted, and the recommended stride frequency for the user is determined directly based on the motion data. In such cases, the user can be informed of his or her recommended stride frequency in a shorter period of time, thereby further improving the motion efficiency.

In some embodiments, the recommended stride frequency for the user is determined based on the motion data. In some embodiments, the recommended stride frequency for the user is determined based on a stride frequency recommendation model. In some embodiments, inputs to the stride frequency recommendation model include, but are not limited to, various types of motion data at a preset stride frequency, for example, any one or a combination of the EMG signals, the posture signals, the ECG signals, the respiratory signals, the pressure signals, etc. An output of the stride frequency recommendation model is the recommended stride frequency. In some embodiments, the stride frequency recommendation model is a trained machine learning model. In some embodiments, the stride frequency recommendation model is pre-trained by a processing device and stored in a storage device. The processing device accesses the storage device to obtain the stride frequency recommendation model.

In some embodiments, the stride frequency recommendation model is obtained by a training process performed based on sample information. The sample information includes the motion data from professionals (e.g., fitness trainers) and/or non-professionals while exercising. In some embodiments, the sample information also includes historical motion data of the user. For example, the user wears the wearable device and performs multiple running motions at different stride frequencies, and the data collection device is used to obtain the motion data, which is used as historical motion data. When using the motion data as the sample information, the physiological state of the user is monitored while obtaining the motion data. In some embodiments, the motion data in the sample information is processed signals (e.g., through segmentation, burr processing, conversion processing, etc.). In some embodiments, the motion data is used as the input to the machine learning model to train the machine learning model. In some embodiments, feature information corresponding to the motion data is used as the input to the machine learning model to train the machine learning model. For example, frequency information and amplitude information of the EMG signals are used as the input to the machine learning model. As another example, the angular speed, an angular speed direction/ an angular speed acceleration value of the posture signals are used as the inputs to the machine learning model. In some embodiments, the machine learning model includes one or more of a linear classification model (LR), a support vector machine model (SVM), a naive Bayesian model (NB), a k-nearest neighbor model (KNN), a decision tree model (DT), an integration model (RF/GDBT, etc.), etc. In some embodiments, when training the machine learning model, the sample information of the different motion data is labeled. Taking the motion data being the EMG signals as an example, the EMG signal generated by the user moving at a first stride frequency is labeled as "the first stride frequency." The EMG signal generated by the user moving at a second stride frequency is labeled as "the second stride frequency." Different EMG signals correspond to different physiological states, and the labeled sample information (i.e., each labeled EMG signal) is used as the input to the machine learning model to train the machine learning model, so as to obtain the stride frequency recommendation model for determining the recommended stride frequency. The recommended stride frequency is output when inputting the motion data and/or the feature information to the machine learning model.

In some embodiments, when obtaining the sample information (i.e., the motion data at different stride frequencies) used to train the machine learning model, the motion speed of the user is constant. In some embodiments, when obtaining the sample information (i.e., the motion data at different stride frequencies) used to train the machine learning model, the motion speed of the user is different. At this point, the motion speed while obtaining the motion data is monitored, and an effect brought by different motion speeds is compensated in the data processing process.

In some embodiments, to improve the accuracy of the recommended stride frequency, the processing device determines the recommended stride frequency of the user based on two or more of the posture signals, the EMG signals, the mechanical signals, the ECG signals, the respiratory signals, the sweat signals, etc. That is, one or more of the posture signals, the EMG signals, the mechanical signals, the ECG signals, the respiratory signals, the sweat signals, etc., and the motion speed of the user, etc., are used as the inputs to the machine learning model to train the machine learning model, which makes the recommended stride frequency output by the machine learning model more suitable for the user. In some embodiments, in practical applications, the recommended stride frequency (also referred to as an initial recommended stride frequency) under a target training task is estimated based on information input by the user. The user first moves at the initial recommended stride frequency, and the actual motion data of the user is collected during the motion process. The system for processing motion data iteratively updates the stride frequency recommendation model based on the actual motion data, so that the stride frequency recommendation model outputs a more accurate recommended stride frequency. In some embodiments, in a long-term use of the user, the system for processing motion data collects more motion data, thereby making the recommended stride frequency recommended by the system for processing motion data more reasonable.

In some embodiments, embodiments of the present disclosure also provide the wearable device. The wearable device includes a wearing body provided with at least one sensor used to obtain the motion data of the user, and a processor configured to perform the method for processing the motion data described in FIG. 3-FIG. 6. In some embodiments, the wearing body includes, but is not limited to, one or more of a top, pants, a belt, socks, knee pads, wrist pads, etc. The sensor includes one or more of an EMG sensor (one or more electrodes), a posture sensor (e.g., a velocity sensor, an inertial sensor, an angular velocity sensor, etc.), a pressure sensor, etc. When the user wears the wearable device and runs, the sensor is in contact with the body portions of the user, so that the motion data is collected while the user is moving. For example, the EMG sensor includes one or more electrodes, and the one or more electrodes, when located at the wearable device corresponding to a muscle portion of the human body, collect the EMG signals of the muscles of the corresponding portion. As another example, the one or more electrodes, when located on the wearable device corresponding to the abdomen of the human body, collect the ECG signals (or the respiratory signals) of the user during motion. As a further example, the posture sensor located at the position of the wearable device corresponding to a joint portion of the human body, such as a knee, an elbow, or an ankle joint, collects the posture signals of the user during motion. More descriptions regarding the wearable device and the method for processing motion data performed by the processor may be found in the related descriptions of FIG. 1- FIG. 6.

In some embodiments, the electrodes provided on the wearing body in contact with the skin of the user are utilized to provide stimulation to the body portion of the user. In some embodiments, the electrodes are utilized to provide the stimulation to the body portions of the user to provide feedback to the user on the recommended stride frequency. In some embodiments, the frequency at which the electrodes stimulate the body portion (i.e., a stimulation frequency) is the same as the recommended stride frequency, and the user adjusts the real-time stride frequency based on the stimulation received by the body until the stride frequency is the same as the stimulation frequency. In some embodiments, the electrodes are also utilized to stimulate the body portion of the user at a dynamic stimulation frequency, a changing trend of the dynamic stimulation frequency is the same as the stride frequency adjustment trend. For example, when the stride frequency adjustment trend is the frequency decrease trend, the initial frequency of the dynamic stimulation frequency is the real-time stride frequency of the user, the target frequency of the dynamic stimulation frequency is the recommended stride frequency, and the dynamic stimulation frequency gradually decreases from the initial frequency to the target frequency. In this process, the stride frequency of the user and the dynamic stimulation frequency are consistent, and the user follows the dynamic stimulation frequency to gradually adjust the stride frequency to the recommended stride frequency. In some embodiments, the adjustment process of the dynamic stimulation frequency changes uniformly. In some embodiments, the adjustment process of the dynamic stimulation frequency changes non-uniformly.

The basic concepts have been described above, and it is apparent to those skilled in the art that the foregoing detailed disclosure serves only as an example and does not constitute a limitation of the present disclosure. Although not explicitly stated here, those skilled in the art may make various modifications, improvements and amendments to the present disclosure. These alterations, improvements, and modifications are intended to be suggested by the present disclosure, and are within the spirit and scope of the exemplary embodiments thereof.

Moreover, certain terminology has been used to describe embodiments of the present disclosure. As in "an embodiment," "one embodiment," and/or " some embodiments" means a feature, a structure, or a characteristic associated with at least one embodiment of the present disclosure. Accordingly, it should be emphasized and noted that two or more references to "one embodiment" or "the embodiment" in different positions in the present disclosure are not intended to be used in the present disclosure. In addition, some features, structures, or characteristics in one or more embodiments of the present disclosure may be appropriately combined.

Furthermore, it will be appreciated by those skilled in the art that aspects of the present disclosure may be illustrated and described by a number of patentable categories or circumstances, including any new and useful process, machine, product, or combination of substances, or any of their new and useful improvements. Accordingly, all aspects of the present disclosure may be performed entirely by hardware, may be performed entirely by software (including firmware, resident software, microcode, etc.), or may be performed by a combination of hardware and software. The above hardware or software may be referred to as "data block," "module," "engine," "unit," "component," or "system." In addition, aspects of the present disclosure may appear as a computer product located in one or more computer-readable media, the product including computer-readable program codes.

Computer storage media may contain propagated data signals with a computer program encoded within it, e.g., on a baseband or as a portion of a carrier. The propagation signals have a variety of manifestations, including an EMG form, an optical form, etc., or suitable combinations thereof. The computer storage medium may be any computer-readable medium, other than a computer-readable storage medium, which is used by connecting to an instruction-executing system, device, or apparatus for communicating, propagating, or transmitting for use. The program code disposed on the computer storage medium is disseminated via any suitable medium, including a radio, a cable, a fiber optic cable, an RF, etc., or a combination thereof.

The computer program code required for the operation of each portion of the present disclosure is written in any one or more programming languages, including object-oriented programming languages such as Java, Scala, Smalltalk, Eiffel, JADE, Emerald, C++, C#, VB.NET, Python, etc., conventional procedural programming languages such as C, Visual Basic, Fortran 2003, Perl, COBOL 2002, PHP, ABAP, dynamic programming languages such as Python, Ruby and Groovy, or other programming languages. The program code can be run entirely on the user's computer, or as a stand-alone package on the user's computer, or partially on the user's computer and partially on a remote computer, or entirely on a remote computer or processing device. In the latter case, the remote computer can be connected to the user's computer through any form of network, such as a local area network (LAN) or wide area network (WAN), or connected to an external computer (e.g., via the Internet), or in a cloud computing environment, or used as a service such as software as a service (SaaS).

Additionally, unless expressly stated in the claims, the order of the processing elements and sequences described herein, the use of numerical letters, or the use of other names are not intended to qualify the order of the processes and methods of the present disclosure. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, while the above-described system components may be implemented by a hardware device, it is also possible to be implemented by a software-only solution, such as installing the described system on an existing processing device or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that the present disclosure object requires more features than the features mentioned in the claims. Rather, claimed subject matter may lie in less than all features of a single foregoing disclosed embodiment.

Some embodiments use numbers to describe the number of components, attributes, and it should be understood that such numbers used in the description of the embodiments are modified in some examples by the modifiers "about," "approximately," or "substantially." Unless otherwise noted, the terms "about," "approximately," or "substantially" indicates that a ±20% variation in the stated number is allowed. Correspondingly, in some embodiments, the numerical parameters used in the present disclosure and the claims are approximations, which can change depending on the desired characteristics of individual embodiments. In some embodiments, the numerical parameters should take into account the specified number of valid digits and employ a general place-keeping. While the numerical domains and parameters used in some embodiments of the present disclosure to confirm the breadth of their ranges are approximations, in specific embodiments such values are set to be as precise as possible within a feasible range.

For each of the patents, patent applications, patent application disclosures, and other materials cited in the present disclosure, such as articles, books, specifications, publications, documents, etc., the entire contents of which are hereby incorporated herein by reference. Except for application history documents that are inconsistent with or conflict with the contents of the present disclosure, and except for documents (currently or hereafter appended to the present disclosure) that limit the broadest scope of the claims of the present disclosure. It should be noted that to the extent that there is an inconsistency or conflict between the descriptions, definitions, and/or use of terminology in the materials appurtenant to the present disclosure and those set forth herein, the descriptions, definitions and/or use of terminology in the present disclosure shall prevail.

At last, it should be understood that the embodiments described in the present disclosure are merely illustrative of the principles of the embodiments of the present disclosure. Other modifications that may be employed are within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A method for processing motion data, comprising:
obtaining motion data of a user at different stride frequencies, the motion data at least including electromyographic (EMG) signals;
determining a physiological state of the user based on the motion data; and
determining a recommended stride frequency for the user based on the physiological state.

2. The method of claim 1, wherein the obtaining the motion data of a user at different stride frequencies includes:
determining a target speed interval; and
obtaining the motion data at different stride frequencies within the target speed interval based on the target speed interval.

3. The method of claim 1, wherein the physiological state includes a muscle efficiency, a muscle tension, and a muscle fatigue, and the determining a recommended stride frequency for the user based on the physiological state includes:
determining a correspondence between physiological states and stride frequencies based on at least one of the muscle efficiency, the muscle tension, and the muscle fatigue; and
determining the recommended stride frequency for the user based on the correspondence.

4. The method of claim 3, wherein the determining a physiological state of the user based on the motion data includes:
determining at least one of the muscle efficiency, the muscle tension, and the muscle fatigue of the user based on the EMG signals.

5. The method of claim 1, wherein the motion data includes electrocardio (ECG) signals and/or posture signals, and the determining a recommended stride frequency for the user based on the physiological state includes:
determining a correspondence between physiological states and stride frequencies based on the ECG signals and/or the posture signals; and
determining the recommended stride frequency for the user based on the correspondence.

6. The method of claim 5, wherein the determining the physiological state of the user based on the motion data includes:
determining the physiological state of the user based on the ECG signals and/or the posture signals, the physiological state including an injury risk for the motion of the user.

7. The method of claim 1, wherein the obtaining motion data of a user at different stride frequencies includes:
obtaining a real-time stride frequency and real-time motion data of the user;
dividing the real-time stride frequency into a plurality of stride frequency intervals; and
determining, based on the plurality of stride frequency intervals, motion data within each different stride frequency interval from the real-time motion data.

8. The method of claim 7, wherein the real-time stride frequency is obtained by an inertial sensor, or the real-time stride frequency is obtained from the EMG signals.

9. The method of claim 1, further including: feeding back the recommended stride frequency to the user.

10. The method of claim 9, wherein the feeding back the recommended stride frequency to the user includes:
obtaining a real-time stride frequency of the user;
determining a stride frequency adjustment trend for the user based on a frequency difference between the real-time stride frequency and the recommended stride frequency; and
guiding the user based on the stride frequency adjustment trend until a stride frequency of the user is the same as the recommended stride frequency.

11. The method of claim 9, wherein the feeding back the recommended stride frequency to the user includes:
feeding back a beat that is the same as the recommended stride frequency to the user based on a preset feedback manner.

12. **The** method of claim 11, wherein the preset feedback manner includes at least one of a voice feedback, a vibration feedback, a light feedback, and a display feedback.

13. **The** method of claim 9, wherein the feeding back the recommended stride frequency to the user includes:
stimulating a body portion of the user based on a biofeedback manner to feed back the recommended stride frequency to the user, wherein the stimulation has a stimulation frequency same as the recommended stride frequency.

14. **The** method of claim 1, wherein the obtaining motion data of a user at different stride frequencies includes:
obtaining a plurality of sets of motion data of the user at a plurality of preset stride frequencies, each of the plurality of sets of motion data corresponding to one of the plurality of preset stride frequencies.

15. The method of claim 14, wherein the obtaining a plurality of sets of motion data of the user at a plurality of preset stride frequencies includes:
obtaining physiological information of the user, the physiological information including body information and motion information; and
determining the plurality of sets of motion data of the user at the plurality of preset stride frequencies based on the physiological information.

16. A system for processing motion data, comprising:
an obtaining module configured to obtain motion data of a user at different stride frequencies, the motion data at least including electromyographic (EMG) signals;
a processing module configured to determine a physiological state of the user based on the motion data; and
a recommendation module configured to determine a recommended stride frequency for the user based on the physiological state.

17. The system of claim 16, wherein the obtain motion data of a user at different stride frequencies includes:
determining a target speed interval; and
obtaining the motion data at different stride frequencies within the target speed interval based on the target speed interval.

18. The system of claim 16, wherein the physiological state includes a muscle efficiency, a muscle tension, and a muscle fatigue, and the determine a recommended stride frequency for the user based on the physiological state includes:
determining a correspondence between physiological states and stride frequencies based on at least one of the muscle efficiency, the muscle tension, and the muscle fatigue; and
determining the recommended stride frequency for the user based on the correspondence.

19. The system of claim 16, wherein the determine a physiological state of the user based on the motion data includes:
determining at least one of the muscle efficiency, the muscle tension, and the muscle fatigue of the user based on the EMG signals.

20. The system of claim 16, wherein the motion data includes electrocardio (ECG) signals and/or posture signals, and the determining a recommended stride frequency for the user based on the physiological state includes:
determining a correspondence between physiological states and stride frequencies based on the ECG signals and/or the posture signals; and
determining the recommended stride frequency for the user based on the correspondence.

21. The system of claim 20, wherein the determining the physiological state of the user based on the motion data includes:
determining the physiological state of the user based on the ECG signals and/or the posture signals, the physiological state including an injury risk for the motion of the user.

22. The system of claim 16, wherein the obtaining motion data of a user at different stride frequencies includes:
obtaining a real-time stride frequency and real-time motion data of the user;
dividing the real-time stride frequency into a plurality of stride frequency intervals; and
determining, based on the plurality of stride frequency intervals, motion data within each different stride frequency interval from the real-time motion data.

23. The system of claim 22, wherein the real-time stride frequency is obtained by an inertial sensor, or the real-time stride frequency is obtained from the EMG signals.

24. The system of claim 16, further including a feedback module configured to feed back the recommended stride frequency to the user.

25. The system of claim 24, wherein the feeding back the recommended stride frequency to the user includes:
obtaining a real-time stride frequency of the user;
determining a stride frequency adjustment trend for the user based on a frequency difference between the real-time stride frequency and the recommended stride frequency; and
guiding the user based on the stride frequency adjustment trend until a stride frequency of the user is the same as the recommended stride frequency.

26. The system of claim 24, wherein the feeding back the recommended stride frequency to the user includes:
feeding back a beat that is the same as the recommended stride frequency to the user based on a preset feedback manner.

27. The system of claim 26, wherein the preset feedback manner includes at least one of a voice feedback, a vibration feedback, a light feedback, and a display feedback.

28. The system of claim 24, wherein the feeding back the recommended stride frequency to the user includes:
stimulating a body portion of the user based on a biofeedback manner to feed back the recommended stride frequency to the user, wherein the stimulation has a stimulation frequency same as the recommended stride frequency.

29. The system of claim 16, wherein the obtain motion data of a user at different stride frequencies includes:
obtaining a plurality of sets of the motion data of the user at a plurality of preset stride frequencies, each of the plurality of sets of motion data corresponding to one of the plurality of preset stride frequencies.

30. The system of claim 29, wherein the obtaining a plurality of sets of motion data of the user at a plurality of preset stride frequencies includes:
obtaining physiological information of the user, the physiological information including body information and motion information; and
determining the plurality of sets of motion data of the user at the plurality of preset stride frequencies based on the physiological information.

31. A wearable device comprising:
a wearing body provided with at least one sensor, the at least one sensor being configured to obtain motion data of a user; and
a processor configured to perform the method of any one of claims 1 to 15.

32. The wearable device of claim 31, wherein the wearing body is further provided with electrodes contacting a skin of the user, and the electrodes are configured to provide stimulation to a body portion of the user.
